(19) Europäisches Patentamt
European Patent Office
Office européen des brevets

(11) EP 0 894 497 B1

(12) **EUROPÄISCHE PATENTSCHRIFT**

(45) Veröffentlichungstag und Bekanntmachung des Hinweises auf die Patenterteilung:
**31.03.2004 Patentblatt 2004/14**

(51) Int Cl.[7]: **A61K 31/427**, A61K 31/497, C07D 417/04, A61P 29/00

(21) Anmeldenummer: **98112974.5**

(22) Anmeldetag: **13.07.1998**

# (54) Verwendung substituierter Imidazolidin-2,4-dion-Verbindungen als Schmerzmittel

Use of substituted imidazolidin-2,4-diones as analgesics

Utilisation des imidazolidin-2,4-diones substitués comme analgésiques

(84) Benannte Vertragsstaaten:
**AT BE CH CY DE DK ES FI FR GB GR IE IT LI LU MC NL PT SE**
Benannte Erstreckungsstaaten:
**LT LV SI**

(30) Priorität: **31.07.1997 DE 19732928**

(43) Veröffentlichungstag der Anmeldung:
**03.02.1999 Patentblatt 1999/05**

(73) Patentinhaber: **Grünenthal GmbH**
**52078 Aachen (DE)**

(72) Erfinder:
- **Zimmer, Oswald, Dr.**
  **52146 Würselen (DE)**
- **Selve, Norma, Dr.**
  **52078 Aachen (DE)**

(56) Entgegenhaltungen:
**EP-A- 0 770 613** **DE-A- 19 609 847**

- **PATENT ABSTRACTS OF JAPAN vol. 4, no. 85 (C-015), 18. Juni 1980 & JP 55 051068 A (KYOWA HAKKO KOGYO CO LTD), 14. April 1980**
- **PATENT ABSTRACTS OF JAPAN vol. 97, no. 11, 28. November 1997 & JP 09 176131 A (FUJIREBIO INC), 8. Juli 1997**
- **SATSANGI R K ET AL: "1-(4-SUBSTITUTED-THIAZOL-2-YL)HYDANTOINS AS ANTI-INFLAMMATORY AND CNS-ACTIVE AGENTS" PHARMAZIE, Bd. 38, Mai 1983, Seite 341/342 XP002022929**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 894 497 B1

## Beschreibung

Die Erfindung betrifft die Verwendung substituierter Imidazolidin-2,4-dion-Verbindungen zur Herstellung von Arzneimitteln für die Behandlung von Schmerzen.

Schmerz ist ein subjektives Sinneserlebnis, das aus einer sensorischen und einer affektiven Komponente besteht. Die physiologischen Aspekte der Schmerzentstehung umfassen die Rezeption eines jeden physikalischchemischen Reizes in potentiell gewebebedrohender Intensität mit Aktivierung der sogenannten Nociceptoren, speziellen uni- oder polymodalen Nocisensoren hochschwelliger primärer aszendierender Nervenbahnen. Bei der Betrachtung der pathophysiologischen Aspekte der Schmerzentstehung können generell alle Anteile des nociceptiven Systems verändert sein: die Rezeption durch die Nocisensoren, die Weiterleitung auf spinaler Ebene, die Wahrnehmung, Bewußtwerdung und Verarbeitung auf supraspinaler Ebene. Zu plastischen Veränderungen und Chronifizierungen kann es durch Störungen im afferenten System, aber auch durch gestörte Wahrnehmung und Verarbeitung sowie durch Störungen im deszendierenden, kontrollierenden, endogenen schmerzhemmenden System kommen. Bei chronischem oder neuropathischem Schmerz kommt es unter anderem zur Sensibilisierung der Nocisensoren durch endogene oder exogene Substanzen. Bei anhaltendem Reizeinfluß oder durch Störung der Integrität des Nociceptors kann es dann auf spinaler Ebene zu sekundärer und auch zentraler Sensibilisierung kommen. Bekannt sind solche Phänomene insbesondere bei chronischen Entzündungsvorgängen z.B. bei rheumatoider Arthritis. Die im Bereich des Nocisensors von lokalen Entzündungszellen ausgeschütteten entzündungsfördernden Gewebshormone und Transmitter (Histamin, Serotonin, Prostaglandine, Interleukin 1) können zur Sensibilisierung der Nociceptoren mit erniedrigter Reizschwelle und erhöhter Spontanaktivität führen, wobei anhaltende Entzündungsvorgänge mit permanentem Reizeinstrom letzlich zum Aufschaukeln adaptiver Prozesse auch auf spinaler und supraspinaler Ebenen führen.

Substituierte Imidazolidin-2,4-dion-Verbindungen und deren immunmodulierende Wirkung sind aus der deutschen Patentanmeldung 19540027.5 bekannt. Überraschenderweise wurde nun gefunden, daß diese Verbindungen darüber hinaus eine antinociceptive Wirkung besitzen, die sich aus der bisher bekannten immunmodulierenden und antivaskulitischen Wirkung nicht ableiten läßt.

Gegenstand der Erfindung ist dementsprechend die Verwendung von substituierten Imidazolidin-2,4-dion-Verbindungen der Formel I

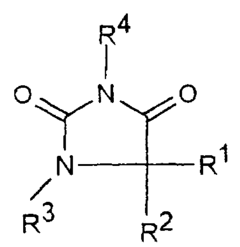

in der $R^1$ $C_{1-6}$-Alkyl oder $C_{3-6}$-Cycloalkyl und $R^2$ $C_{1-6}$-Alkyl, Phenyl, $-(CH_2)_{1-3}$-Phenyl oder $-(CH_2)_{1-4}-COOR^5$ bedeutet oder $R^1$ und $R^2$ zusammen $-(CH_2)_{4-6}-$, $-(CH_2)_2-O-(CH_2)_2-$ oder

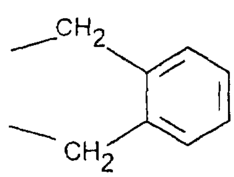

bedeuten, $R^3$ H, $C_{1-5}$-Alkyl oder $-(CH_2)_{1-4}-COOR^5$ bedeutet, $R^4$ ein Heteroaromat aus der Gruppe mit den Formeln

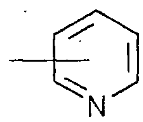, 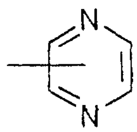, 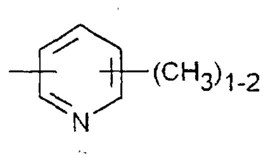, 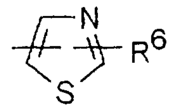,

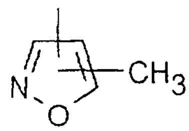, 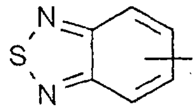, 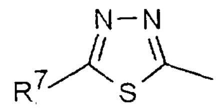

ist, $R^5$ $C_{1-3}$-Alkyl darstellt, $R^6$ H, $C_{1-4}$-Alkyl, Phenyl oder Benzyl bedeutet und $R^7$ H, $C_{1-4}$-Alkyl oder Trifluormethyl bedeutet, zur Herstellung eines Arzneimittels für die Behandlung von Schmerzen.

Substituierte Imidazolidin-2,4-dion-Verbindungen der Formel I mit $R^1$ $C_{1-6}$-Alkyl, $R^2$ $C_{1-6}$-Alkyl, Phenyl oder $-(CH_2)_{1-3}$-Phenyl oder $R^1$ und $R^2$ zusammen $-(CH_2)_{4-6}$oder

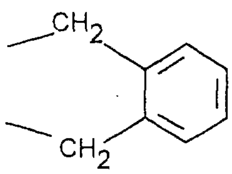

und $R^3$ H oder $C_{1-5}$-Alkyl werden bevorzugt und Verbindungen der Formel I mit $R^1$ $C_{1-4}$-Alkyl, $R^2$ $C_{3-5}$-Alkyl oder Phenyl oder $R^1$ und $R^2$ zusammen $-(CH_2)_5$- oder

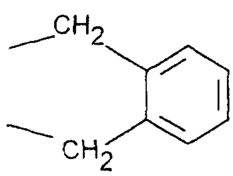

und $R^3$ H oder $C_{1-4}$-Alkyl besonders bevorzugt verwendet. Insbesondere bevorzugt ist der Einsatz von substituierten Imidazolidin-2,4-dion-Verbindungen der Formel I mit $R^4$ Thiazol-2-yl oder Pyrazin-2-yl.

Wenigstens eine substituierte Imidazolidin-2,4-dion-Verbindung der Formel I kann als einziger Wirkstoff oder in Kombination mit ein oder mehreren weiteren Wirkstoffen zur Herstellung eines Schmerzmittels eingesetzt werden. Besonders geeignete weitere Wirkstoffe sind ausgewählt aus mindestens einer der Gruppen Opioide, Tramadol-Material und nichtopioiden Schmerzmitteln. Beispiele für Opioide sind Morphin, Hydromorphon, Codein, Oxycodon, Dihydrocodein, Dextropropoxyphen, Buprenorphin, Levomethadon, Fentanyl, Sufentanil sowie die pharmazeutischen Salze der vorgenannten Wirkstoffe. Tramadol-Material umfaßt Tramadol [(1RS; 2RS)-2-[(Dimethylamino)methyl]-1-(3-methoxyphenyl) cyclohexanol)], Tramadol-N-oxyd, O-Demethyltramadol, die in den deutschen Patentanmeldungen DE 4426245, 195 25 137.7, 196 09 847.5 und 197 10 628.5 offenbarten Tramadolabkömmlinge sowie die pharmazeutischen Salze der vorgenannten Tramadol-Materialien in racemischer oder enantiomerer Form. Als nichtopioide Schmerzmittel eignen sich beispielsweise saure nichtopioide Carbonsäuren und Carbonsäureester, wie Salicylate, Arylessigsäuren und Arylpropionsäuren, beispielsweise Acetylsalicylsäure, Diclofenac, Naproxen, Ketoprofen und Ibuprofen, saure nichtopioide heterozyklische Ketoenolsäuren wie Oxicame und Pyrazolidindione, beispielsweise Piroxicam und Tenoxicam, nichtsaure nichtopioide Aniline und Pyrazolinone, beispielsweise Paracetamol und Metamizol,

sowie nichtopioide Pyridylcarbamate, beispielsweise Flupirtin und Benzoxazocine, beispielsweise Nefopam.

Bei erfindungsgemäßer Verwendung einer substituierten Imidazolidin-2,4-dion-Verbindung der Formel I in Kombination mit einem weiteren Schmerzmittel liegt das Gewichtsverhältnis der beiden Wirkstoffe üblicherweise zwischen 1:0,01 und 1:25.

Die schmerzhemmende Wirkung der erfindungsgemäß zu verwendenden Substanzen ist unabhängig davon, ob der Schmerz von einer Entzündung verursacht wird. Darüber hinaus korreliert die antinociceptive Wirkung der erfindungsgemäß zu verwendenden Substanzen nicht mit der TNF$\alpha$-Hemmung. Die antinociceptive Wirkung der substituierten Imidazolidin-2,4-dion-Verbindungen der Formel I läßt sich nicht mit bekannten antinociceptiven Mechanismen, wie $\mu$-Opioidrezeptoragonismus, monoaminerge Reuptake-Hemmung oder mit einer Interaktion mit einem Rezeptor wie Adenosin, $\alpha/\beta$-Adrenoceptor, GABA, Galanin, Glutamat/NMDA, Histamin, Somatostatin, Tachykinin, VIP oder NPY, oder mit einem der folgenden Kanäle Calcium, Kalium, MAO, NO-Synthetase, Proteinkinase oder Enzym/second messenger system erklären. Es ist nicht auszuschließen, daß die schmerzhemmende Wirkung auf eine Wechselwirkung mit endogenem NGF (Nerve Growth Factor) zurückzuführen ist.

Substituierte Imidazolidin-2,4-dion-Verbindungen der Formel I werden vorzugsweise zur Herstellung von Arzneimitteln für die Behandlung chronischer Schmerzzustände verwendet. Chronische Schmerzzustände, d.h. chronisch entzündliche und chronisch neuropathische Schmerzzustände treten beispielsweise bei Rheuma, sekundär entzündlicher Osteoarthrose, Rückenschmerzen, Spannungskopfschmerzen, Traumata, Herpes Zoster und Trigeminus neuralgie auf.

Zur Herstellung der Schmerzmittel werden die erfindungsgemäß zu verwendenden Verbindungen zusammen mit Trägermaterialien, Füllstoffen, Lösungsmitteln, Verdünnungsmitteln, Farbstoffen und/oder Bindemitteln eingesetzt. Die Auswahl der Hilfsstoffe sowie die einzusetzenden Mengen hängen davon ab, ob das herzustellende Schmerzmittel oral, intravenös, intraperitoneal, intradermal, intramuskulär, intranasal, buccal oder lokal appliziert werden soll. Für die orale Applikation eignen sich Zubereitungen in Form von Matrixtabletten, überzogenen Tabletten, Mehrschichttabletten, Kautabletten, Dragees, Kapseln, Granulaten, Tropfen, Säften oder Sirupen, für die parenterale topische und inhalative Applikation Lösungen, Suspensionen, leicht rekonstituierbare Trockenzubereitungen sowie Sprays. Erfindungsgemäße Verbindungen in einem Depot in gelöster Form, einer Trägerfolie oder einem Pflaster ggf. unter Zusatz von die Hautpenetration fördernden Mitteln sind Beispiele für geeignete perkutane Applikationsformen. Aus oral oder perkutan anwendbaren Zubereitungsformen können die erfindungsgemäßen Verbindungen verzögert freigesetzt werden.

Üblicherweise werden pro Dosiseinheit 5 bis 500 mg, vorzugsweise 10 bis 200 mg wenigstens einer substituierten Imidazolidin-2,4-dion-Verbindung der Formel I eingesetzt.

**Beispiele**

Die schmerzhemmende Wirkung der erfindungsgemäß zu verwendenden Substanzen wurde an drei Testmodellen bestimmt. Folgende substituierte Imidazolidin-2,4-dion-Verbindungen wurden nach dem in der deutschen Patentanmeldung 19540027.5 beschriebenen Verfahren eingesetzt:

5-Ethyl-5-phenyl-3-pyrazin-2-yl-imidazolidin-2,4-dion (Verbindung 1)
5-Ethyl-5-phenyl-3-thiazol-2-yl-imidazolidin-2,4-dion (Verbindung 2)
3-Thiazol-2-yl-1,3-diaza-spiro [4.5] decan-2,4-dion (Verbindung 3)
5-Isobutyl-5-methyl-3-thiazol-2-yl-imidazolidin-2,4-dion (Verbindung 4)
3-Thiazol-2-yl-1,3-diaza-spiro [4.4] benzononan-2,4 dion (Verbindung 5)
1-Propyl-3-thiazol-2-yl-1,3-diaza-spiro [4.5] decan-2,4-dion (Verbindung 6)
5,5-Dipropyl-3-thiazol-2-yl-imidazolidin-2,4-dion (Verbindung 7)

**1.Randall-Selitto-Test**

Zur Bestimmung der antinociceptiven in vivo Wirkung im Randall-Selitto-Test (Arch. Int. Pharmacodyn. Ther. 111, 409 (1957)) wurde durch Injektion von 0,1 ml einer 20%igen Bäckerhefe-Suspension, injiziert in eine Rattenhinterpfote, ein Ödem induziert, das nach 4 Stunden zu einer ausgeprägten Mechanohyperalgesie führte. Durch zunehmenden Druck (0-450 g/mm$^2$) mit einem Stempel (0,2 mm Spitzendurchmesser) auf die entzündete Rattenhinterpfote wurde anschließend ein Schmerz erzeugt und als Meßwert der Druck, bei dem die Vokalisationsreaktion der Ratte erfolgte, bestimmt. Tiere, die bis zum maximal zugelassenen Druck nicht vokalisiert hatten, galten als voll analgetisch. Die Gabe der Prüfsubstanzen erfolgte intraperitoneal in einer Dosierung von 10 ml/kg 5 und 30 Minuten vor der Randall-Selitto-Messung. Die Versuchsergebnisse sind als MPE (maximum possible effect) in %, nach der Formel:

$$[\text{Nachsubstanzmeßwert - Vorsubstanzmeßwert}]/[\text{Maximalwert - Vorsubstanzmeßwert}] \times 100$$

dargestellt.

$ED_{50}$-Berechnungen erfolgten nach linearer Regression, Vertrauensbereiche wurden nach Litchfield und Wilcoxon berechnet (J. Pharmacol. Exp. Ther. 95, 99 (1949)).

Tabelle: Antinociceptive Wirkung im Randall-Selitto-Test

| Substanz | Dosis mg/kg | Antinociceptive in vivo-Wirkung | |
|---|---|---|---|
| | | MPE (%) | $ED_{50}$ (mg/kg) |
| Vehikel (wäßrige Carboxymethyl-cellulose-sus-pension) | - | - 15,6 ± 7,4 | |
| Vergleich Acetylsalicylsäure | 464 | 36,3 ± 8,2 | |
| Verbindung 1 | 100 | 10,2 ± 2,9 | |
| Verbindung 2 | | | 46,8 (37,8 - 58,2) |
| Verbindung 3 | | | 71,0 (58,6 - 87,2) |
| Verbindung 4 | 100 | 15,1 ± 7,0 | |
| Verbindung 5 | 100 | 35,0 ± 16,7 | |
| Verbindung 6 | | | 70,1 (55,9 - 90,9) |
| Verbindung 7 | | | 128,7 (97,5 - 152,6) |
| Vehikel (wäßrige Carboxymethylcellulose-suspension) | | 2,3 - 19,8 | |

| | | | |
|---|---|---|---|
| Verbindung 3 | 46,4 | 14,5 ± 5,96 | |
| Tramadol | 2,15 | 13,5 ± 3,45 | |
| Verbindung 3 und Tramadol | 46,4 und 2,15 | 47,9 ± 8,77 | |
| Morphin | 1,46 | 12,0 ± 4,56 | |
| Verbindung 3 und Morphin | 46,4 und 1,46 | 23,3 ± 4,84 | |
| Metamizol | 21,5 | 5,1 ± 3,86 | |
| Verbindung 3 und Metamizol | 46,4 und 21,5 | 41,5 ± 11,52 | |
| Acetylsalicylsäure (ASS) | 464 | 1,1 ± 3,68 | |
| Verbindung 3 und ASS | 46,4 und 464 | 43,0 ± 8,28 | |
| Paracetamol | 464 | 19,7 ± 3,03 | |
| Verbindung 3 und Paracetamol | 46,4 und 464 | 26,9 ± 6,45 | |

**2. Formalin-Test**

Zur Überprüfung indikationsbezogener Wirksamkeit bei chronischen Schmerzen wurde der Formalin-Test (Pain 4, 161 (1977); Pain 30, 103 (1987)) ausgewählt. Hierbei wurden nach Injektion von Formalin in eine Hinterpfote von Ratte oder Maus die Schmerzreaktionen des Tieres als komplexe Verhaltensmuster durch Beobachtung erfaßt und für die Wirksamkeitsprüfung der Verbindung 3 nach einem Score-System quantifiziert. Dieser Test bleibt nicht auf den Nachweis spinaler Fluchtreaktionen oder supraspinal gesteuerter Einzelreaktionen beschränkt, sondern erfaßt das komplex veränderte Verhalten des Gesamttieres.

Für Verbindung 3 wurde bei der Ratte ein $ED_{50}$-Wert von 35,6 mg/kg für die direkte analgetische Wirkung und ein $ED_{50}$-Wert von 33,3 mg/kg für die analgetische Hemmung in der zweiten chronischen Phase und bei der Maus ein $ED_{50}$-Wert von 31,8 mg/kg für die direkte analgetische Wirkung und ein $ED_{50}$-Wert von 32,8 mg/kg für die analgetische Hemmung in der zweiten chronischen Phase ermittelt.

**3. Hot-Plate-Test**

Zur Überprüfung der Wirkung bei akutem, nichtentzündungsbedingtem thermischen Reiz wurde die spinale/supraspinale nociceptive Reaktionszeit in der Hot-Plate-Versuchsanordnung untersucht (J. Pharmacol. Exp. Ther. 133, 400 (1944)). Für Verbindung 3 wurde bei der Maus ein $ED_{50}$-Wert von ca. 100 mg/kg i.p. für die direkte Antithermohyperalgesiewirkung bestimmt.

## Patentansprüche

**1.** Verwendung von substituierten Imidazolidin-2,4-dion-Verbindungen der Formel I

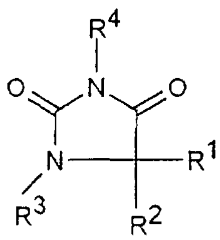

in der $R^1$ $C_{1-6}$-Alkyl oder $C_{3-6}$-Cycloalkyl und $R^2$ $C_{1-6}$-Alkyl, Phenyl, $-(CH_2)_{1-3}$-Phenyl oder $-(CH_2)_{1-4}-COOR^5$ bedeutet oder $R^1$ und $R^2$ zusammen $-(CH_2)_{4-6}-$, $-(CH_2)_2-O-(CH_2)_2-$ oder

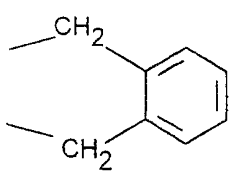

bedeuten, $R^3$ H, $C_{1-5}$-Alkyl oder $-(CH_2)_{1-4}-COOR^5$ bedeutet, $R^4$ ein Heteroaromat aus der Gruppe mit den Formeln

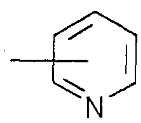 , 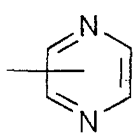 , 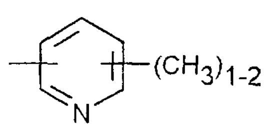 , 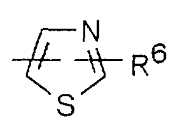 ,

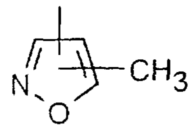
,
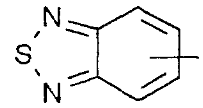
,
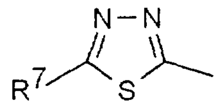

ist, $R^5$ $C_{1-3}$-Alkyl darstellt, $R^6$ H, $C_{1-4}$-Alkyl, Phenyl oder Benzyl bedeutet und $R^7$ H, $C_{1-4}$-Alkyl oder Trifluormethyl bedeutet, zur Herstellung eines Arzneimittels für die Behandlung von Schmerzen.

2. Verwendung nach Anspruch 1 **dadurch gekennzeichnet, daß** substituierte Imidazolidin-2,4-dion-Verbindungen der Formel I mit $R^1$ $C_{1-6}$-Alkyl, $R^2$ $C_{1-6}$-Alkyl, Phenyl oder $-(CH_2)_{1-3}$-Phenyl oder $R^1$ und $R^2$ zusammen -(CH2)4-6- oder

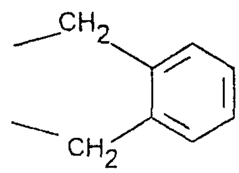

und $R^3$ H oder $C_{1-5}$-Alkyl verwendet werden.

3. Verwendung nach einem oder beiden der Ansprüche 1 bis 2 **dadurch gekennzeichnet, daß** substituierte Imidazolidin-2,4-dion-Verbindungen der Formel I mit $R^1$ $C_{1-4}$-Alkyl, $R^2$ $C_{3-5}$-Alkyl oder Phenyl oder $R^1$ und $R^2$ zusammen $-(CH_2)_5$- oder

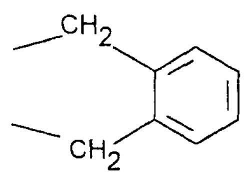

und $R^3$ H oder $C_{1-4}$-Alkyl verwendet werden.

4. Verwendung nach einem oder mehreren der Ansprüche 1 bis 3 **dadurch gekennzeichnet, daß** substituierte Imidazolidin-2,4-dion-Verbindungen der Formel I mit $R^4$ Thiazol-2-yl oder Pyrazin-2-yl verwendet werden.

5. Verwendung nach einem oder mehreren der Ansprüche 1 bis 4 **dadurch gekennzeichnet, daß** mindestens eine substituierte Imidazolidin-2,4-dion-Verbindung der Formel I zusammen mit mindestens einem Wirkstoff ausgewählt aus mindestens einer der Gruppen Opioide, Tramadol-Material und nichtopioide Schmerzmittel verwendet werden.

6. Verwendung nach einem oder mehreren der Ansprüche 1 bis 5 **dadurch gekennzeichnet, daß** substituierte Imidazolidin-2,4-dion-Verbindungen der Formel I zur Herstellung eines Arzneimittels für die Behandlung chronischer Schmerzen verwendet werden.

## Claims

1. Use of substituted 2,4-imidazolidinedione compounds corresponding to the formula I

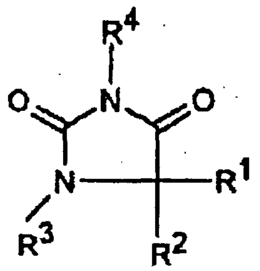

wherein $R^1$ denotes $C_1$-$C_6$-alkyl or $C_{3-6}$-cycloalkyl, and $R^2$ denotes $C_{1-6}$-alkyl, phenyl, $-(CH_2)_{1-3}$-phenyl or $-(CH_2)_{1-4}$-$COOR^5$, or $R^1$ and $R^2$ together denote $-(CH_2)_{4-6}$-, $-(CH_2)_2$-O-$(CH_2)_2$- or

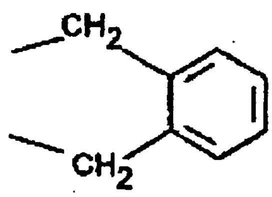

$R^3$ denotes H, $C_{1-5}$-alkyl or $-(CH_2)_{1-4}$-$COOR^5$, $R^4$ is a heteroaromatic compound selected from the group having the formulae

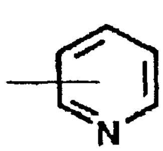
,
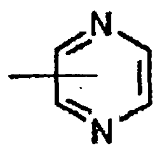
,
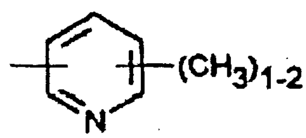
,
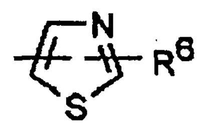
,

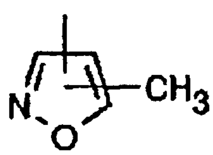
,
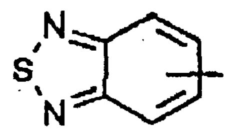
,
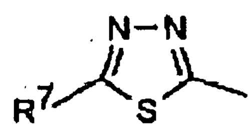

$R^5$ denotes $C_{1-3}$ alkyl, $R^6$ denotes H, $C_{1-4}$-alkyl, phenyl or benzyl, and $R^7$ denotes H, $C_{1-4}$-alkyl or trifluoromethyl, for the production of a pharmaceutical preparation for the treatment of pain.

2. Use according to claim 1, **characterised in that** substituted 2,4-imidazolidinedione compounds corresponding to the formula I are used with $R^1$ $C_{1-6}$-alkyl, $R^2$ $C_{1-6}$-alkyl, phenyl or $-(CH_2)_{1-3}$-phenyl, or $R^1$ and $R^2$ together $-(CH_2)_{4-6}$- or

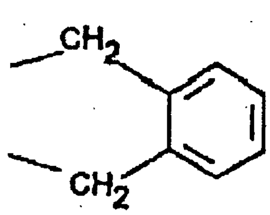

and $R^3$ H or $C_{1-5}$-alkyl.

3. Use according to either one or both of claims 1 and 2, **characterised in that** substituted 2,4-imidazolidinedione

compounds corresponding to the formula I are used with $R^1$ $C_{1-4}$-alkyl, $R^2$ $C_{3-5}$-alkyl or phenyl, or $R^1$ and $R^2$ together $-(CH_2)_5-$ or

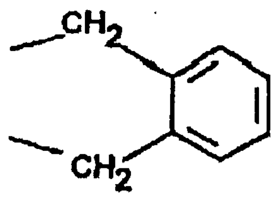

and $R^3$ H or $C_{1-4}$-alkyl.

4. Use according to one or more of claims 1 to 3, **characterised in that** substituted 2,4-imidazolidinedione compounds corresponding to the formula I are used with $R^4$ thiazol-2-yl or pyrazin-2-yl.

5. Use according to one or more of claims 1 to 4, **characterised in that** at least one substituted 2,4-imidazolidinedione compound corresponding to the formula I is used together with at least one active substance selected from at least one of the groups comprising opioids, tramadol material and non-opioid analgesics.

6. Use according to one or more of claims 1 to 5, **characterised in that** substituted 2,4-imidazolidinedione compounds corresponding to the formula I are used for the production of a pharmaceutical preparation for the treatment of chronic pain.

**Revendications**

1. Utilisation d'imidazolidine-2,4-diones substituées de formule I

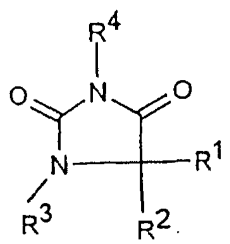

dans laquelle $R^1$ désigne un reste alkyle en $C_1$ à $C_6$ ou cycloalkyle en $C_3$ à $C_6$ et $R^2$ désigne un reste alkyle en $C_1$ à $C_6$, phényle, $-(CH_2)_{1à3}$-phényle ou $-(CH_2)_{1à4}$-$COOR^5$, ou bien $R^1$ et $R^2$ forment ensemble un reste $-(CH_2)_{4à6}$-, $-(CH_2)_2$-O-$(CH_2)_2$- ou

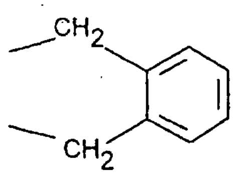

$R^3$ représente H, un reste alkyle en $C_1$ à $C_5$ ou $-(CH_2)_{1à4}$-$COOR^5$, $R^4$ est un reste hétéroaromatique choisi dans le groupe répondant aux formules

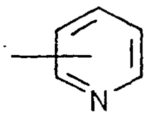 , 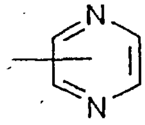 , 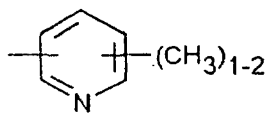 , 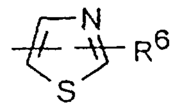 ,

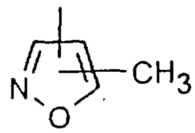 , 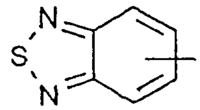 , 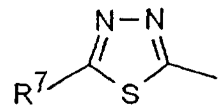

$R^5$ est un reste alkyle en $C_1$ à $C_3$, $R^6$ représente H, un reste alkyle en $C_1$ à $C_4$, phényle ou benzyle et $R^7$ représente H, un reste alkyle en $C_1$ à $C_4$ ou un reste trifluorométhyle, pour la préparation d'un médicament destiné au traitement de douleurs.

2. Utilisation suivant la revendication 1, **caractérisée en ce qu'**on utilise des imidazolidine-2,4-diones substituées de formule I dans laquelle $R^1$ est un reste alkyle en $C_1$ à $C_6$, $R^2$ est un reste alkyle en $C_1$ à $C_6$, phényle ou $-(CH_2)_{1 à 3}$-phényle ou bien $R^1$ et $R^2$ forment ensemble un reste $-(CH_2)_{4 à 6}$- ou

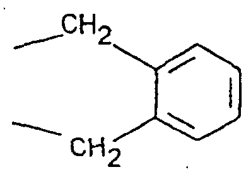

et $R^3$ représente H ou un reste alkyle en $C_1$ à $C_5$.

3. Utilisation suivant l'une des revendications 1 et 2 ou les deux, **caractérisée en ce qu'**on utilise des imidazolidine-2,4-diones substituées de formule I, dans laquelle $R^1$ est un reste alkyle en $C_1$ à $C_4$, $R^2$ est un reste alkyle en $C_3$ à $C_5$ ou phényle ou bien $R^1$ et $R^2$ forment ensemble un reste $-(CH_2)_5$- ou

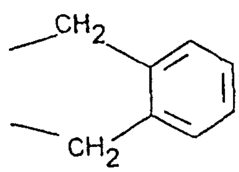

et $R^3$ représente H ou un reste alkyle en $C_1$ à $C_4$.

4. Utilisation suivant l'une ou plusieurs des revendications 1 à 3, **caractérisée en ce qu'**on utilise des imidazolidine-2,4-diones substituées de formule I dans laquelle $R^4$ est un reste thiazole-2-yle ou pyrazine-2-yle.

5. Utilisation suivant l'une ou plusieurs des revendications 1 à 4, **caractérisée en ce qu'**on utilise au moins une imidazolidine-2,4-dione substituée de formule I conjointement avec au moins une substance active choisie dans l'un au moins des groupes comprenant des opioïdes, une substance du type du tramadol et des antalgiques non opioïdes.

6. Utilisation suivant l'une ou plusieurs des revendications 1 à 5, **caractérisée en ce qu'**on utilise des imidazolidine-2,4-diones substituées de formule I pour la préparation d'un médicament destiné au traitement de douleurs chroniques.